# EUROPEAN PATENT APPLICATION

(11) **EP 4 361 285 A1**
(43) Date of publication of application: **01.05.2024**
(21) Application number: 22203173.4
(22) Date of filing: 24.10.2022
(51) Int. Cl.: C12Q 1/6841

(54) **OPTICAL DECODING FOR DETECTION OF ROLONY IN TISSUE OR CELLS**

(71) Applicant: Miltenyi Biotec B.V. & Co. KG, 51429 Bergisch Gladbach (DE)
(72) Inventor: MEYER, Hansueli, 51429 Bergisch Gladbach (DE); PINARD, Robert, 51429 Bergisch Gladbach (DE)
(74) Representative: Kisters, Michael Marcus

(57) **Abstract**

The invention is directed to a method for determining the spatial localisation and a sequence of interest of at least a part of at least one RNA strand comprising the steps
a. providing a plurality of oligonucleotides comprising a unit complementary to the sequence of interest and at least one unit comprising 5 to 30 nucleotides as detection sequences.
b. hybridizing the oligonucleotides to the at least one RNA strand via the sequence of interest and removing unhybridized oligonucleotides
**characterized in**
c. providing a plurality of detection probes comprising a first fluorescence dye and a first detection oligonucleotide which is at least in part complementary to at least one detection sequence in the oligonucleotides and hybridizing the detection probes to the at least one detection sequences of the oligonucleotide
d. removing unhybridized detection probes
e. detecting the detection probes thereby determining the sequence of interest and the spatial localisation of the RNA strands on the surface.

## Description

### BACKGROUND

The present invention is directed to a process for spatial decoding of m-RNA molecules on a tissue or cell(s).

Many spatial multiomics applications trying to detect analytes on cells or tissue by using fluorescence labeling. The number of detectable signals coming from fluorescently labeled analytes is often limited by the number of available fluorescence colors of the instrument. Therefore methods were developed to overcome the limitations of available fluorescence colors.

Analyte decoding can be performed by doing multiple rounds of fluorescence labeling and assigning a molecular code to each detectable analyte. This method is described in US 10227639 and US 11021737 by assigning a color code to different genes. The method requires multiple cycles for decoding.

On way to enable multiplexing fluorescence signals is to use padlock methods which are for example disclosed in "Highly multiplexed subcellular RNA sequencing in situ" by Lee etal., Science. 2014 March 21; 343(6177): 1360-1363. doi:10.1126/science.1250212 or "Efficient In Situ Detection of mRNAs using the Chlorella virus DNA ligase for Padlock Probe Ligation" by Nils Schneider and Matthias Meier; February 5, 2020 -Cold Spring Harbor Laboratory Press

A comprehensive assay for targeted multiplex amplification of human DNA sequences is published by Sujatha Krishnakumar et al.; PNAS sent for review February 19, 2008.

Further, WO2017143155A2 discloses multiplex alteration of cells using a pooled nucleic acid library and analysis thereof and WO2018045181A1 discloses Methods of generating libraries of nucleic acid sequences for detection via fluorescent in situ sequencing.

The published padlock methods allow sequencing of DNA or RNA, but do not give any information from what specific cell or tissue location the sequenced DNA or RNA origins from.

### SUMMARY

Object of the invention is a method for determining the spatial localisation and a sequence of interest of at least a part of at least one RNA strand comprising the steps
a. providing a plurality of oligonucleotides comprising a unit complementary to the sequence of interest and at least one unit comprising 5 to 30 nucleotides as detection sequences.
b. hybridizing the oligonucleotides to the at least one RNA strand via the sequence of interest and removing unhybridized oligonucleotides
   **characterized in**
c. providing a plurality of detection probes comprising a first fluorescence dye and a first detection oligonucleotide which is at least in part complementary to at least one detection sequence in the oligonucleotides and hybridizing the detection probes to the at least one detection sequences of the oligonucleotide
d. removing unhybridized detection probes
e. detecting the detection probes thereby determining the sequence of interest and the spatial localisation of the RNA strands on the surface.

### BRIEF DESCRIPTION OF THE DRAWINGS

Fig. 1 shows exemplary a decoding table for each targeted gene based on 4 colors in one cycle.

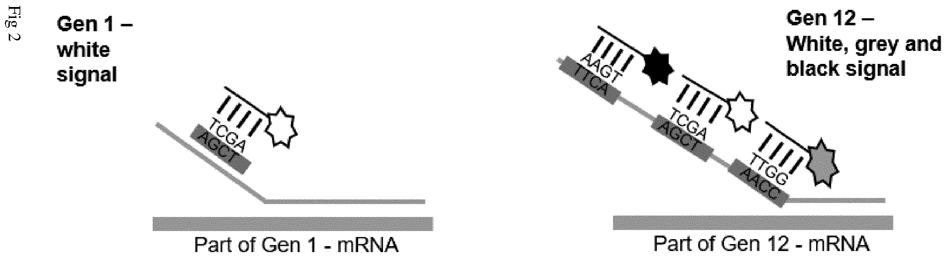

Fig. 2 shows a oligonucleotide binding to a first targeted gene with one color and another oligonucleotide binding to a another targeted gene with three color to be detected according to the table in Fig. 1

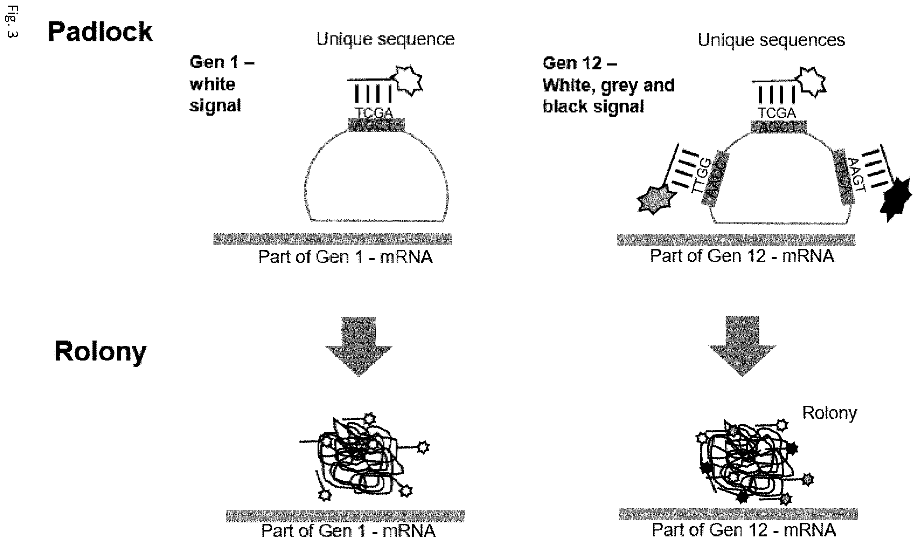

Fig. 3 shows in the upper part a padlock-shaped oligonucleotide binding to a first targeted gene with one color and another padlock-shaped oligonucleotide binding to a another targeted gene with three color to be detected according to the table in Fig. 1. The lower part refers to the same padlock-shaped oligonucleotides, but after RCA.

### DETAILED DESCRIPTION

Many spatial multiomics applications trying to detect analytes on cells or tissue by using fluorescence labeling. The number of detectable signals coming from fluorescently labeled analytes is often limited by the number of available fluorescence colors of the instrument. Therefore methods were developed to overcome the limitations of available fluorescence colors.

The oligonucleotides may comprises 1 to 10 detection sequences having the same or a different sequence and wherein a corresponding number of detection probes comprising same or different fluorescence dyes are provided. This enables multiplexing detection.

In a variant of the invention, after detection, the hybridized detection probes are removed.

The oligonucleotides may be linear or padlock-shaped, i.e. have a circular structure.

In the embodiment using padlock-shaped oligonucleotides, the oligonucleotide is hybridized with its 5' and 3' ends to complementary parts of the at least one RNA strand; wherein the 5' and 3' ends of the hybridized oligonucleotide are combined with each other to create a single strand circular template hybridized to the at least one RNA strand and wherein the detection probes are hybridized to the single strand circular template.

In a variant thereof, the oligonucleotide is hybridized with its 5' and 3' ends to such complementary parts of the at least one RNA strand that the 5' and 3' ends of the oligonucleotide are hybridized adjacent to each other and wherein 5' and 3' ends of the oligonucleotide are ligated with each other.

In both cases, the single strand circular template is multiplied by a polymerase capable of rolling circle amplification into a plurality of concatemers thereby forming a rolony.

The present method combines the use of oligonucleotide forming a padlock while hybridized (padlock probes) and hybridization for detection of bound probes and sequencing targeted portion of RNA or DNA transcript at a cellular level with less limitation in the amount of transcripts and the length of the sequence that can be analyzed. In this approach, padlock probes containing one or more barcode regions in their core are utilized both as FISH probes and also to capture RNA portion that can be sequenced.

Padlock probes have proven very successful in polymerizing short portion of nucleic acids to which it has been hybridized to. Most padlock approaches begin by reverse transcribing the target into cDNA. Alternatively, the padlock can also be hybridized to cDNA which would require additional steps that could be bypassed by targeting the mRNA directly. This technology is known.

Finally, the circularized padlock probes are used as a template for rolling circle amplification (RCA) to generate a DNA strand used for sequencing. The thus obtained DNA strands are hereinafter referred to as "rolonies" or "DNA nanoballs".

The detection probes used in the method of the present invention may by comprised of an oligonucleotide with 2 or more nucleotides capable of binding to at least a part of the barcode region and a detection region selected from the group comprising a magnetic particle, a fluorescence dye, a radioactive label. Preferable, the detection probes comprise fluorescent dyes known from the art of immunofluorescence technologies, e.g., flow cytometry or fluorescence microscopy.

As mentioned the padlock contains one or more barcode regions in their core utilized as FISH probes. Each padlock is designed with a specific barcode region able to bind one or more fluorescently labeled (FISH) probe. Each targeted gene on the tissue or cell has its own assigned specific barcode region and if labeled with fluorescence probes lighting up in different detectable colors. Those detectable colors are all collected during one cycle when the chemistry is applied. The order of taking the color images can be random. The order does not matter. In addition compared to other methods all the needed information is collected during one cycle. Each color signal from each rolony during this one cycle is extracted and recorded. Afterwards a color code for each rolony is determined and correlated with a predetermined targeted gene.

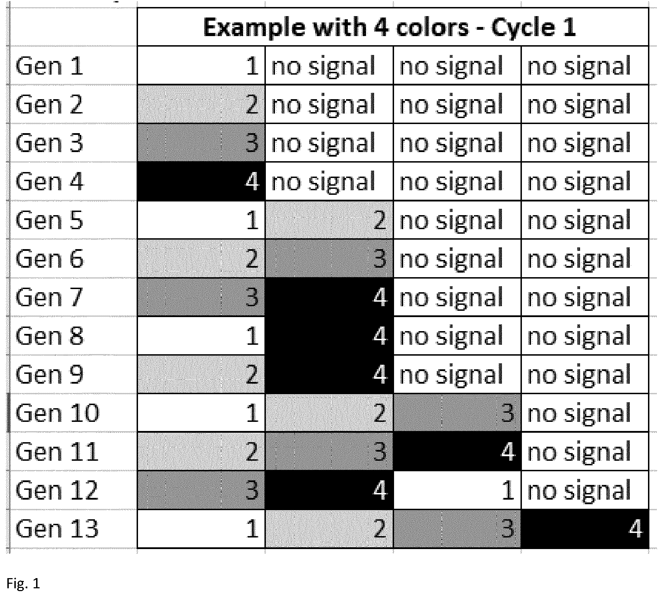

The table in Fig. 1 gives an overview in case of a four color system (1-4, , white, grey, dark grey and black) and shows how each gene has its own color code detected during one cycle.

## Claims

1. A method for determining the spatial localisation and a sequence of interest of at least a part of at least one RNA strand comprising the steps
a. providing a plurality of oligonucleotides comprising a unit complementary to the sequence of interest and at least one unit comprising 5 to 30 nucleotides as detection sequences.
b. hybridizing the oligonucleotides to the at least one RNA strand via the sequence of interest and removing unhybridized oligonucleotides
**characterized in**
c. providing a plurality of detection probes comprising a first fluorescence dye and a first detection oligonucleotide which is at least in part complementary to at least one detection sequence in the oligonucleotides and hybridizing the detection probes to the at least one detection sequences of the oligonucleotide
d. removing unhybridized detection probes
e. detecting the detection probes thereby determining the sequence of interest and the spatial localisation of the RNA strands on the surface.

2. Method according to claim 1 **characterized in that** the oligonucleotides comprises 1 to 10 detection sequences having the same or a different sequence and wherein a corresponding number of detection probes comprising same or different fluorescence dyes are provided.

3. Method according to claim 1 or 2 **characterized in that** after detection, the hybridized detection probes are removed.

4. Method according to any of the claims 1 to 3 **characterized in that** the oligonucleotide is hybridized with its 5' and 3' ends to complementary parts of the at least one RNA strand; wherein the 5' and 3' ends of the hybridized oligonucleotide are combined with each other to create a single strand circular template hybridized to the at least one RNA strand and wherein the detection probes are hybridized to the single strand circular template.

5. Method according to claim 4 **characterized in that** the oligonucleotide is hybridized with its 5' and 3' ends to such complementary parts of the at least one RNA strand that the 5' and 3' ends of the oligonucleotide are hybridized adjacent to each other and wherein 5' and 3' ends of the oligonucleotide are ligated with each other.

6. Method according to claim 4 or 5 **characterized in that** the single strand circular template is multiplied by a polymerase capable of rolling circle amplification into a plurality of concatemers thereby forming a rolony.

7. Method according to any of the claims 1 to 7 **characterized in that** the oligonucleotides comprises 1 to 200 detection sequences having the same or a different sequence and wherein a corresponding number of detection probes comprising same or different fluorescence dyes are provided.

8. Method according to any of the claims 1 to 7 **characterized in that** the at least one oligonucleotide is provided with at least one primer region for RCA.

9. Method according to any of the claims 1 to 8 **characterized in that** the at least one RNA strand on a surface is provided from a plurality of cells or from a tissue section and wherein before or after step a), the sample is lysed and permeabilized on a surface.
